Europäisches Patentamt

European Patent Office

Office européen des brevets

(11) Veröffentlichungsnummer: **0 139 861**

**A1**

(12) **EUROPÄISCHE PATENTANMELDUNG**

(21) Anmeldenummer: **84108124.3**

(22) Anmeldetag: **11.07.84**

(51) Int. Cl.⁴: **A 61 M 1/00**

(30) Priorität: **28.07.83 DE 3327217**

(43) Veröffentlichungstag der Anmeldung:
**08.05.85 Patentblatt 85/19**

(84) Benannte Vertragsstaaten:
**AT BE CH DE FR GB IT LI LU NL SE**

(71) Anmelder: **Azizoglu, Mustafa Ayhan, Dr.**
**Westring 69**
**D-6231 Schwalbach/Ts.(DE)**

(72) Erfinder: **Azizoglu, Mustafa Ayhan, Dr.**
**Westring 69**
**D-6231 Schwalbach/Ts.(DE)**

(74) Vertreter: **Gudel, Diether, Dr. et al,**
**Patentanwälte Dr. V. Schmied-Kowarzik Dipl.-Ing. G.**
**Dannenberg Dr. P. Weinhold Dr. D. Gudel Dipl.-Ing. S.**
**Schubert Dr. P. Barz Grosse Eschenheimer Strasse 39**
**D-6000 Frankfurt am Main 1(DE)**

(54) **Vorrichtung zur Behandlung von Bauchfellentzündungen und dergleichen.**

(57) Beschrieben wird eine Vorrichtung zur Behandlung von Bauchfellentzündungen und anderen Erkrankungen der Bauch höhle, die sich in der Bauchhöhle des menschlichen oder tierischen Körpers abspielen, wobei eine zweiteilige, glockenartige Haube (1, 8) aus starrem Material flüssigkeitsdicht in die betreffende Öffnung eingesetzt wird. Der untere, ringförmige Haubenteil (1) wird an der Haut und an der hinteren Scheide des geraden Bauchmuskels mit Bauchfell vernäht, so daß das Innere der Bauchhöhle nach Entfernen des oberen Haubenteils (8) leicht zugänglich ist. Die Glocke (1, 8) bietet ausreichend Platz für die in der Bauchhöhle befindlichen Organe und es können auch in jeweils gewünschter Anzahl und Anordnung Drains (13, 14) über die Glocke (1, 8) in die Bauchhöhle eingeführt werden.

Fig.2

Beschreibung

Die Erfindung betrifft eine Vorrichtung zur Behandlung von Erkrankungen der Bauchhöhle des menschlichen oder tierischen Körpers mit einem starren Bauteil, gegebenenfalls mit durch das Bauteil hindurchgeführten Drains, das in die geöffnete Bauchhöhle eingesetzt wird, wobei sich die geöffnete Bauchdecke an eine Außenwand des Bauteils anlegt, und mit Mitteln zur Befestigung des Bauteils an der geöffneten Bauchhöhle.

Zur Behandlung von Bauchfellentzündungen und dergleichen wird die Bauchdecke geöffnet und es werden Drains in die Bauchhöhle eingeführt, über die eine geeignete Spülflüssigkeit zugeführt bzw. Körperflüssigkeit und Spülflüssigkeit abgesaugt werden kann. Ein guter Überblick über den aktuellen Stand der Technik bei derartigen Operationen steht in der Zeitschrift "Der Chirurg", 54. Jahrgang, Heft 5, Mai 1983, Seiten 293-311.

Demnach ist es heute üblich, die offene Wunde mit einer Wellendrainage aus Silikon mit schlitzförmigen Öffnungen abzudecken, damit das Sekret aus der Bauchhöhle abfließen kann. Auch wird die Bauchhöhle gelegentlich mit einem mattenartigen Teil aus mehreren, nebeneinander befindlichen Schläuchen abgedeckt, über die dann die Drains zugeführt werden. Nachteilig bei diesen bekannten Behandlungsmethoden ist es aber, daß die unter der Bauchöffnung befindlichen Organe, insbesondere der Darm, nicht genügend Platz zum Ausdehnen haben. Hier ist zu berücksichtigen, daß diese Organe bei einer Bauchfellentzündung sich häufig stark erweitern, so daß dann die Gefahr besteht, daß die einzelnen Darmschlingen miteinander verkleben, weil sie nicht ausreichend Platz zum ungehinderten Ausdehnen finden. Auch ist keine ausreichende Vorsorge dafür getroffen, daß die Bauchöffnung tatsächlich ungehindert frei und offen bleibt.

Die DE-AS 2 754 775 beschreibt eine Vorrichtung mit den eingangs genannten Merkmalen, wobei das Bauteil als vollzylindrischer Körper mit mehreren Durchtrittsöffnungen für die Drains ausgebildet ist. An seine Außenwand ist ein Ringflansch mit Löchern an dessen Umfang angeformt, mit dem das Bauteil nach dem Einsetzen in die geöffnete Bauchhöhle an der Bauchdecke des Patienten angenäht werden kann.

Diese Vorrichtung ist somit lediglich für Bauchhöhlenspülungen geeignet. Sie ist bei Erkrankungen der Bauchhöhle nachteilig, weil der vollzylindrische Körper keinen Platz zum Ausdehnen der erkrankten Organe der Bauchhöhle bietet. Eine mehrmalige Revision der geöffneten Bauchhöhle ist hiermit weder beabsichtigt noch möglich.

Die US-PS 3,026,874 beschreibt eine Wundabdeckung, welche die Beobachtung offener Wunden ermöglicht und Eingriffe in dieselben erlaubt, z.B. durch Spülung, Drainage oder Zufuhr von Medikamenten. Sie besteht im wesentlichen aus einem auf die Haut über der betreffenden Wunde aufgelegten Ring, der über einen Gürtel am Patienten befestigt ist. In den Ring münden Drains ein.

Die US-PS 2,367,690 beschreibt eine durchsichtige, aufklappbare Haube, die mit ihrer Unterseite an einem Ringflansch befestigt ist, der auf eine zu behandelnde Stelle des Patienten aufgeklebt wird. Mit beiden vorstehend beschriebenen bekannten Vorrichtungen ist eine Behandlung von Erkrankungen der Bauchhöhle nicht möglich und wird auch nicht beabsichtigt.

Der Erfindung liegt die Aufgabe zugrunde, eine Vorrichtung zur Behandlung von Erkrankungen der Bauchhöhle der eingangs genannten Art vorzuschlagen, die ausreichend Platz für die unter der Körperöffnung befindlichen Organe schafft so daß die Entzündung medizinisch einwandfrei versorgt werden kann. Auch soll die betreffende Körperöffnung (Bauchöffnung) stets ausreichend und sicher während der Behandlungsdauer offen gehalten werden, um ggfs. wiederholte Untersuchungen und Behandlungen leicht durchführbar zu machen.

Zur Lösung dieser Aufgabe ist die Erfindung dadurch gekennzeichnet, daß das Bauteil als starrer Ring mit auf dem Ring flüssigkeitsdicht und leicht lösbar aufgesetzter, starrer Haube ausgebildet ist, wobei die Mittel zur Befestigung des Bauteils an der geöffneten Bauchhöhle am unteren Rand des Ringes vorgesehen sind.

Nach dem Anlegen des Schnitts wird somit der erwähnte starre Ring in die dadurch gebildete Öffnung eingesetzt und flüssigkeitsdicht mit dem die Öffnung berandenden Gewebe verbunden, beispielsweise vernäht. Auf die obere Kante dieses starren Rings wird eine glockenartige Haube, ebenfalls aus starrem Material, aufgesetzt, wodurch die gesamte Operationswunde von der erfindungsgemäßen Vorrichtung unter Ausbildung eines ausreichenden Volumens innerhalb der Glocke abgedeckt wird. Es wird dadurch ausreichend Platz für Versorgungs- und Entsorgungsleitungen (Drains) geschaffen und auch die jetzt freiliegenden inneren Organe können sich nach oben in die glockenartige Haube ungehindert ausdehnen, so daß beispielsweise die Darmschlingen nicht mehr verkleben. Die abnehmbare Haube sorgt dafür, daß eventuell notwendige Untersuchungen und Behandlungen der Bauchhöhle ohne größeren Aufwand möglich sind. Insgesamt wird durch die erfindungsgemäße Vorrichtung daher eine bedeutend verbesserte medizinische Versorgung und Behandlung gewährleistet.

Es wurde bereits erwähnt, daß der Ring flüssigkeitsdicht in die Öffnung eingesetzt werden muß. Diesbezüglich wird es bevorzugt, wenn nicht nur im unteren Bereich des Ringes Befestigungsmittel für den Ring an der hinteren Scheide des geraden Bauchmuskels mit Bauchfell vorgesehen sind, sondern dazu beabstandet nach oben weitere, obere Befestigungsmittel für die Befestigung des Ringes an der Haut. Der Abstand zwischen den beiden Befestigungsmitteln soll etwa der Dicke der Bauchdecke entsprechen.

Für die erwähnte Befestigung wird es bevorzugt, wenn diese durch Vernähen erfolgt. Hierzu sollen die erwähnten Befestigungsmittel als radial nach außen weisende Flansche mit etwa in axialer Richtung verlaufenden Durchtrittsöffnungen ausgebildet sein. Die Durchtrittsöffnungen dienen dann zur Aufnahme des Nahtmaterials.

Zwischen den beiden Befestigungsringen wird somit eine Rille ausgebildet. Diese kann in vorteilhafter Weise zur Aufnahme von Abdichtungen ausgenutzt werden, beispielsweise durch Umlegen eines Mullstreifens, von weichen Silikonschläuchen oder auch eines ringförmigen, weichen Kissens, das mit Flüssigkeit oder Luft gefüllt ist.

Die Haube kann über ein geeignetes Gelenk klappbar am Ring befestigt sein oder auch als Ganzes vom Ring abgesetzt und wieder aufgesetzt werden können. Sie kann dann einen geeigneten Griff haben.

Der Grundriß des Ringen - und damit auch der aufgesetzten Haube - soll in etwa der Form des betreffenden Schnittes angepaßt sein. Es wird daher ein in etwa elliptischer Grundriß für den Ring bevorzugt. Die Form des Ringes und damit auch der Haube wird derjenigen der Wunde noch besser angepaßt, wenn die schmalen Kanten der Ellipse zugespitzt verlaufen, wie dies ebenfalls bevorzugt wird.

Weiterhin wird es bevorzugt, wenn zumindest die Haube aus durchsichtigem Kunststoffmaterial besteht. Dadurch kann man die offene Bauchhöhle unterhalb der Haube jederzeit beobachten. Man wird ein derartiges Kunststoffmaterial verwenden, welches leicht desinfiziert werden kann, d.h. ein Material mit einer möglichst glatten Oberfläche. Gut eignet sich hierfür Polytetrafluoräthylen. Im allgemeinen wird man den Ring aus demselben Kunststoffmaterial herstellen, obgleich dies nicht unbedingt notwendig ist. Auch der Ring soll aus einem Material bestehen, welches leicht desinfiziert werden kann.

Im Ring und/oder in der Haube soll außerdem wenigstens eine Öffnung für den Durchtritt eines Drains vorgesehen sein. In Ausnahmefällen wird man möglicherweise auch ohne derartige Durchtrittsöffnungen auskommen.

Um die erfindungsgemäße Vorrichtung an unterschiedliche Körpergrößen und Wundgrößen anpassen zu können, wird es bevorzugt, wenn ein Satz von mehreren Ringen unterschiedlicher Größe mit jeweils dazu passenden Hauben vorgesehen ist. Insbesondere ist hierbei an unterschiedliche Abmessungen in der Draufsicht des Ringes gedacht; ggfs. kann der Ring aber auch eine unterschiedliche Höhe haben, um leicht an unterschiedlich dicke Bauchdecken angepaßt werden zu können. Es können auch Ringe - und damit Hauben - in unterschiedlicher Formgebung vorgesehen sein.

Um die erfindungsgemäße Vorrichtung und insbesondere die Haube leicht steril halten zu können, kann man auch eine Schutzhaube mit einem Befestigungsgurt vorsehen, die die Haube unter Abstand umgibt. Dadurch lassen sich innerhalb der Schutzhaube besonders einfach sterile Bedingungen schaffen.

Sollte ggfs. doch Flüssigkeit aus dem Ring nach außen gedrungen sein, so kann diese ggfs. mit Hilfe eines Entwässerungsschlauches abgeleitet werden, an den vorzugsweise eine geeignete Pumpe angeschlossen ist. Der Entwässerungsschlauch hat über seine Länge verteilt mehrere Öffnungen, über die die Flüssigkeit angesaugt wird. Er wird an der Außenwand des Ringes verlegt, und zwar an beiden Seiten des Ringes. Diese beiden Schläuche werden dann getrennt oder über ein gemeinsames Verbindungsstück nach außen geführt.

Die Erfindung wird im folgenden anhand von Ausführungsbeispielen näher erläutert, aus denen sich weitere wichtige Merkmale ergeben. Es zeigt:

Fig. 1 - perspektivisch und schematisch eine Grundform der erfindungsgemäßen Vorrichtung mit Schutzhaube;

Fig. 2 - eine demgegenüber abgeänderte Ausführungsform, wobei in die Haube Drains einmünden;

Fig. 3 - eine weitere, abgeänderte Ausführungsform, wobei in die Haube und/oder in den
Ring Drains einmünden;

Fig. 4 - eine Draufsicht auf den Ring zur Erläuterung seines Profils;

Fig. 5 - eine Draufsicht entsprechend Fig. 4
bei einer anderen, bevorzugten Form des
Ringes;

Fig. 6 - in einer Seitenansicht eine Ausführungsform
ähnlich Fig. 1;

Fig. 7 - einen Mittelschnitt durch den Ring der
Vorrichtung nach Fig. 6;

Fig. 8 - vergrößert einen Schnitt durch einen Spannring,
der den Ring und die Haube miteinander verbindet;

Fig. 9 - schematisch in einer Draufsicht den unteren
Teil des Ringes mit Entwässerungsschläuchen;

Fig. 10 - einen Schnitt durch den unteren linken Teil des
Ringes bei einer weiteren Ausführungsform, wobei der Ring aus zwei teleskopartigen Teilringen
besteht;

Fig. 11 - einen Schnitt durch den unteren Teil des
Ringes mit einer Befestigungsmöglichkeit durch
an einer Klemmleiste befestigte, dübelartige
Fortsätze;

Fig. 12 - eine Draufsicht auf die Klemmleiste nach
Fig. 11.

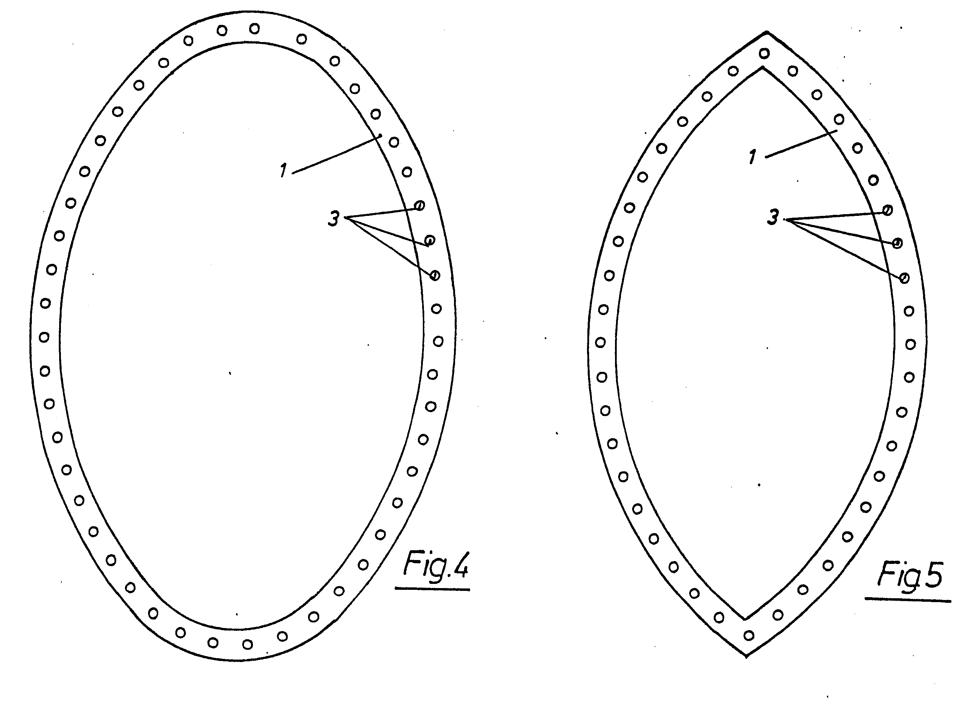

Zunächst sei anhand vonFig. 1 das Prinzip der Erfindung
erläutert. Die dort gezeigte Vorrichtung besteht aus
einem Ring 1 aus starrem Material, vorzugsweise durchsichtigem Polytetrafluoräthylen. Die Form des Ringes kann
man am besten den Fig. 4 oder 5 entnehmen. Der Ring hat
an seiner Unterseite einen ringsum laufenden und radial
nach außen vorstehenden Flansch 2, in dem nebeneinander
eine Vielzahl von in axialer Richtung durchgehenden
Löchern 3 ausgebildet ist. Über den Befestigungsflansch
mit seinen Löchern kann daher der Ring mit dem Bindegewebe vernäht werden.

In einem Abstand 4 von einigen Zentimetern über dem unteren
Flansch 2 befindet sich ein weiterer, oberer Ringflansch
5, ebenfalls mit Durchgangslöchern 6 in im wesentlichen
axialer Richtung. Über diesem oberen Flansch 5 mit seinen
Löchern 6 wird der Ring mit der Haut vernäht. Der Abstand 4
entspricht daher dem üblichen Abstand zwischen dem Bindegewebe und der Haut im Bauchfellbereich.

In einem weiteren Abstand von wenigen Zentimetern über dem
oberen Ringflansch 5 endet der Ring 1 in einer Kante 7.

Eine glockenförmige Haube 8, ebenfalls aus durchsichtigem
Polytetrafluoräthylen, ist flüssigkeitsdicht mit ihrer
unteren Kante 9 auf die Kante 7 des Ringes aufgesetzt.
Zwischen den beiden Kanten 7, 9 befindet sich dabei ein
geeigneter Dichtring. Die Haube ist am Ring leicht lösbar
befestigt, beispielsweise üner mehrere um den Umfang des
Ringes und der Haube verteilte Klammern 10. Es können auch andere bekannte Befestigungsmittel verwendet werden, wobei nur dafür gesorgt werden muß, daß die Haube leicht lösbar und dennoch flüssigkeitsdicht am Ring befestigt wird

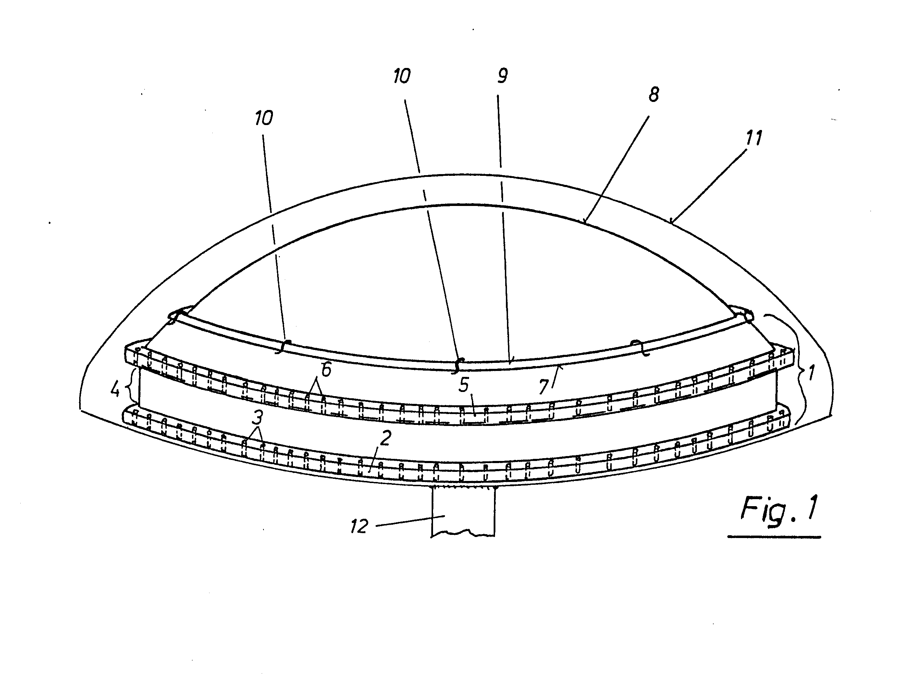

In Fig. 1 ist außerdem gezeigt, daß noch eine Schutzhaube 11, die größer als der Ring 1 mit seiner Haube 8 ist, beabstandet von der Haube 8 diese umgibt. Die Schutzhaube 11 wird beispielsweise mit Hilfe eines Gurtes 12, der um den Leib des Patienten herumgeht, an diesem befestigt.

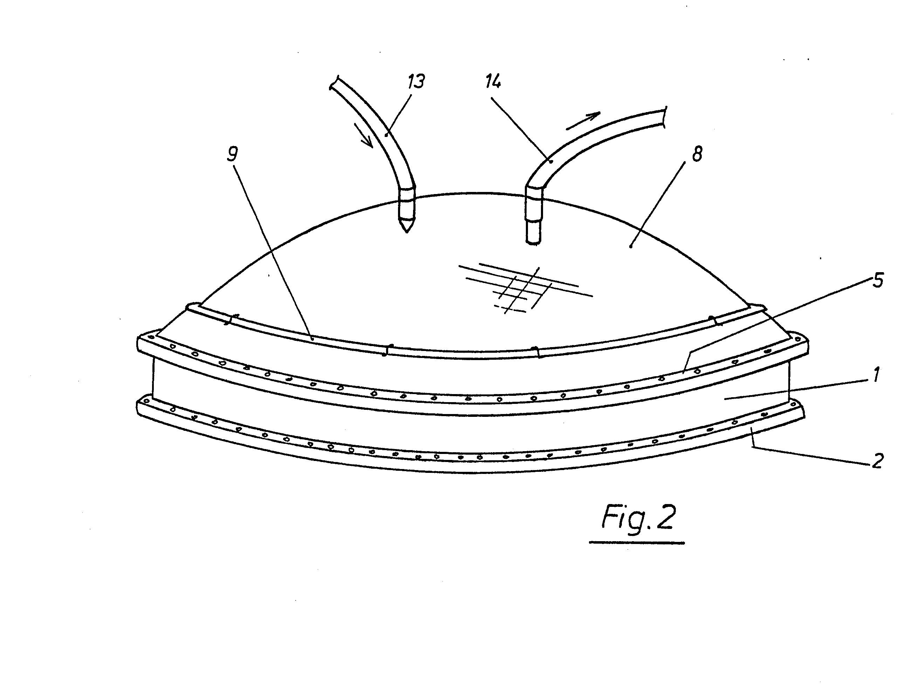

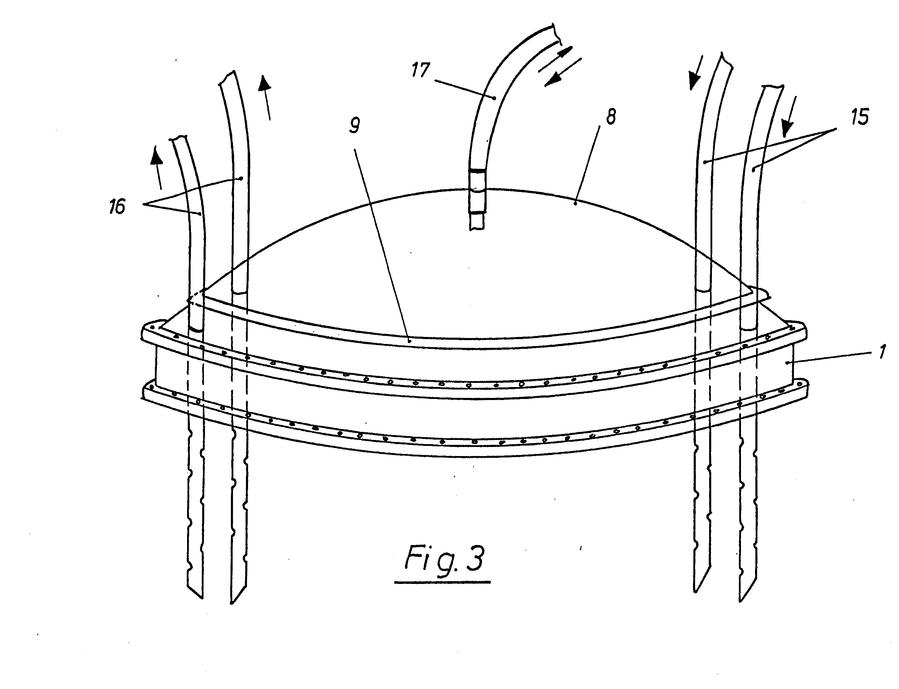

Die Fig. 2 und 3 zeigen mehrere Möglichkeiten der Führung von Drains in die erfindungsgemäße Vorrichtung. Nach Fig. 2 ist ein Drain 13 für die Zufuhr einer Spülflüssigkeit vorgesehen, der im mittleren Teil in die Haube 8 einmündet. Ein weiterer Drain 14 ist für die Abfuhr vorgesehen. Beide Drains reichen entweder, ähnlich wie in Fig. 3 gezeigt, bis in den Bereich des Ringes 1, oder sie enden auch kurz unterhalb der Decke der Haube 8. Bei dieser Ausführungsform wird es bevorzugt, wenn die Drains 13, 14, wie zeichnerisch dargestellt, kurz unterhalb der Haubedecke enden, damit die Haube 8 leicht und ungestört abgehoben und wieder aufgesetzt werden kann.

Bei der Ausführungsform nach Fig. 3 sind statt dessen Drains 15 für die Zufuhr einer Spülflüssigkeit im Ring 1 befestigt und weitere Drains 16 für den Abzug der Flüssigkeit. Diese können, wie zeichnerisch dargestellt, bis unterhalb der Ebene des Ringes 1 reichen.

Zusätzlich oder alternativ kann auch ein einziger Drain 17 in die Haube 8 für die abwechselnde Zufuhr und Abfuhr von Flüssigkeiten einmünden.

Die Fig. 4 und 5 zeigen bevorzugte Formgebungen für den

- 10 -

Ring mitsamt seiner Haube zur optimalen Anpassung an die Form der Öffnung der Bauchdecke zwecks Behandlung einer Bauchfellentzündung. Dabei wird die Form nach Fig. 5 bevorzugt, weil sich die zugespitzten Schmalseiten besser der Form des Operationsschnitts anpassen. Zumindest der untere Flansch 2, vorzugsweise auch der daran nach oben anschließende Teil des Ringes 1, etwa bis zum oberen Flansch 5, soll diese Form haben, nicht notwendigerweise aber auch der obere Rand 7,weil dann eine einfacher geformte Haube 8 verwendet werden kann.

Die Haube 8 hat etwa die Form einer Halbkugel oder eines Kugelabschnitts. Sie bietet zusammen mit dem der Befestigung und Abdichtung dienenden Ring 1 dazu, die Bauchwunde geöffnet zu lassen und Platz zum Ausdehnen entzündeter Organe zu schaffen. Die Haube 8 ist durchsichtig, so daß das Innere der geöffneten Bauchhöhle stets beobachtet werden kann.

Die Drains sind entweder im Ring bzw. in der Haube fest verankert oder auch über selbstdichtende Manschetten eingeführt. Gegebenenfalls kann man auch ohne die Drains auskommen, wie in Fig. 1 gezeigt. Die Anzahl und Anordnung der Drains, z.B. gerader oder schräger Verlauf, um sie sowohl im Oberbauch als auch im Unterbauch gut plazieren zu können, richten sich nach den jeweiligen Anwendungsfällen. Auch kann die Befestigung des Ringes auf andere Art und Weise erfolgen, sofern diese nur flüssigkeitsdicht ist. Gegebenenfalls ist hierzu, wie bereits erwähnt, ein Dichtring zwischen die Flansche 2 und 5 gelegt. Auch kann man einen anderen, bekannten Schnellverschluß für die leicht lösbare Verbindung der Haube mit dem Ring vorsehen.

Die Drains sind ggfs. auch mit Ventilen versehen, um den Durchfluß nur in einer Richtung zu ermöglichen bzw. sie ggfs. schließen zu können. Die Drains sollen mindestens mehrere Zentimeter über den Ring bzw. die Haube vorstehen, um eine gute Anschlußmöglichkeit auszubilden.

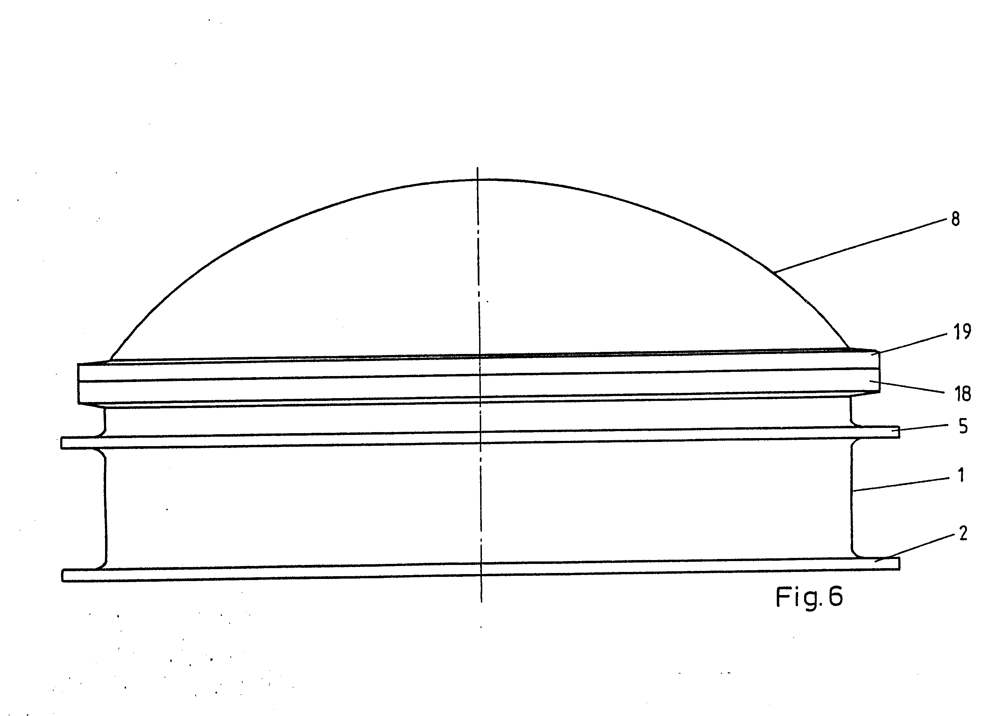

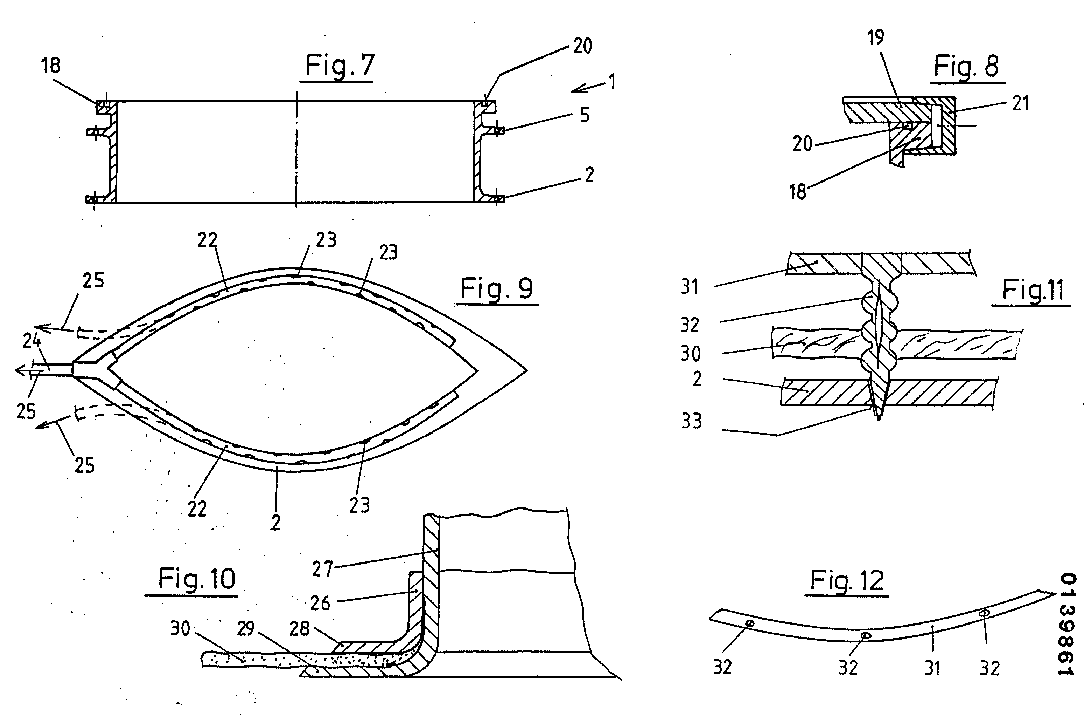

Fig. 6 zeigt, daß der Ring 1 an seinem oberen Ende einen nach außen weisenden Umfangsflansch 18 haben kann, auf dem die Haube 8 mit einem entsprechenden unteren Umfangsflansch 19 aufliegt. Dadurch wird eine gute Dicht- und Verbindungsfläche zwischen beiden Teilen 1, 8 geschaffen. Hierzu vergleiche auch Fig. 7, woraus ebenfalls hervorgeht, daß in der Oberseite des Umfangsflansches 18 des Ringes 1 eine Ringnut 20 zur Aufnahme eines Dichtungsringes eingearbeitet ist.

Fig. 8 zeigt, daß die beiden Flansche 18, 19 gleichzeitig zur leicht lösbaren und dennoch flüssigkeitsdichten Verbindung zwischen den Teilen 1, 8 dienen, nämlich indem ein U-förmig profiliertes Spannband 21 die beiden Flansche 18, 19 umfaßt. Das Spannband wird mit einem geeigneten Verschluß mit einem Hebel geöffnet und verschlossen, wie an sich bekannt.

Nach Fig. 9 können auch noch Entwässerungsschläuche 22 mit über ihre Länge verteilten Löchern 23 zur Aufnahme von dort gegebenenfalls durch den Ring nach außen hindurchgetretener Flüssigkeit an der Außenwand des Ringes 1 verlegt sein. Die beiden Schläuche 22 sind entweder über ein Y-förmiges Verbindungsstück 24 gemeinsam oder auch ohne ein solches Verbindungsstück getrennt an eine Abzugsleitung mit Pumpe angeschlossen, die die dort abgesogene Flüssigkeit in Richtung des Pfeiles 25 fördert.

Fig. 10 zeigt eine alternative Möglichkeit der Befestigung des Gewebes an der Unterseite des Ringes 1. Hierzu besteht der Ring aus zwei teleskopartig ineinander gesetzten Teilringen 26, 27, die flüssigkeitsdicht gegeneinander abgedichtet sind. Beide Ringe haben nach außen reichende Umfangsflansche 28 bzw. 29, zwischen denen das Gewebe 30 rings um den Ring 1 eingeklemmt werden kann.

Die Fig. 11 und 12 zeigen eine weitere Möglichkeit der Befestigung des Gewebes 30 am unteren Bereich des Ringes 1. Hierzu sind zwei Klemmleisten 31 an der rechten und linken Seite des einstückigen Ringes 1 vorgesehen, und zwar beabstandet über dem unteren Flansch 2 des Ringes. An den Klemmleisten sind beabstandet voneinander mehrere dübelartige Fortsätze 32 befestigt, die durch vorher im Gewebe 30 angelegte, enge Löcher hindurchgreifen und in entsprechende Löcher 33 im unteren Flansch 2 eindringen. Dadurch ist es möglich, unterschiedlich dicke Gewebeschichten 30 zwischen dem Flansch 2 und der Klemmleiste 31 zu befestigen und dadurch den Ring 1 flüssigkeitsdicht in der geöffneten Bauchhöhle festzulegen.

Für die flüssigkeitsdichte Befestigung des Ringes in der geöffneten Bauchhöhle und auch für die lösbare Befestigung des Ringes 1 an der Haube 8 können auch andere, hier nicht beschriebene und an sich bekannte Maßnahmen vorgesehen werden. Bezüglich der Profilierung des Ringes, zumindest in seinem unteren Bereich wird das Profil nach Fig. 5 bevorzugt. Auch sollen ein oder mehrere Drains in den Ring bzw. in die Haube einmünden, wie die Fig. 2 und 3 zeigen. Der Ring 1 braucht nicht aus durchsichtigem Material zu bestehen, wohl aber wird dies für die Haube 8 bevorzugt. Am besten handhabbar dürfte die Ausbildung der

0139861

Vorrichtung nach Fig. 6 bis 8 sein mit Profilierung nach Fig. 5 und den Drains nach Fig. 2, 3. Dies ist die bevorzugte Ausführungsform.

Mit der erfindungsgemäßen Vorrichtung können Bauchfellentzündungen, Bauchspeicheldrüsenentzündungen und andere Erkrankungen der Bauchhöhe behandelt werden. Die Verbindungsmittel 5, 6 am oberen Rand des Ringes dienen der Abdichtung zwischen der offenen Bauchhöhle und dem Ring, damit dort keine Kontaminationen entstehen können.

Ansprüche

1. Vorrichtung zur Behandlung von Erkrankungen der Bauchhöhle des menschlichen oder tierischen Körpers mit
einem starren Bauteil, ggf. mit durch das Bauteil
hindurchgeführten Drains (13 - 17), das in die geöffnete
Bauchhöhle eingesetzt wird, wobei sich die geöffnete
Bauchdecke an eine Außenwand des Bauteils anlegt,
und mit Mitteln (2, 3, 5, 6) zur Befestigung des Bauteils
an der geöffneten Bauchhöhle,
d a d u r c h   g e k e n n z e i c h n e t,
daß das Bauteil als starrer Ring (1) mit auf den
Ring (1) flüssigkeitsdicht und leicht lösbar aufgesetzter
starrer Haube (8) ausgebildet ist, wobei die Mittel
(2, 3) zur Befestigung des Bauteils an der geöffneten
Bauchhöhle am unteren Rand des Ringes (1) vorgesehen
sind.

2. Vorrichtung nach Anspruch 1,
d a d u r c h   g e k e n n z e i c h n e t,
daß an der Außenseite des Ringes (1) und in einem Abstand
(4) über den unteren Befestigungsmitteln (2, 3), der etwa
der Dicke der Bauchdecke entspricht, Mittel (5, 6) zur
Befestigung des Ringes (1) an der Haut vorgesehen sind.

3. Vorrichtung nach Anspruch 1 oder 2,
d a d u r c h   g e k e n n z e i c h n e t,
daß die Befestigungsmittel (2, 3, 5, 6) als radial nach
außen weisende Flansche mit etwa in axialer Richtung verlaufenden Durchtrittsöffnungen ausgebildet sind.

4. Vorrichtung nach einem der Ansprüche 1 bis 3,
d a d u r c h  g e k e n n z e i c h n e t ,
daß zur Abdichtung des Ringes (1) in der Öffnung um diesen
ein Dichtring gelegt ist.

5. Vorrichtung nach einem der Ansprüche 1 bis 4,
d a d u r c h  g e k e n n z e i c h n e t ,
daß der Ring (1) im Grundriß zumindest an seiner Unterseite
etwa elliptisch ist, wobei die schmalen Kanten der
Ellipse zugespitzt verlaufen.

6. Vorrichtung nach einem der Ansprüche 1 bis 5,
d a d u r c h  g e k e n n z e i c h n e t ,
daß ein U-förmig profiliertes Spannband vorgesehen ist,
das übereinander liegende Außenflansche des Ringes und
der Haube umgibt.

7. Vorrichtung nach einem der Ansprüche 1 bis 6,
d a d u r c h  g e k e n n z e i c h n e t ,
daß ein Entwässerungsschlauch mit über seiner Länge
vorgesehenen Öffnungen an der Außenwand des Ringes vorgesehen ist.

8. Vorrichtung nach einem der Ansprüche 1 bis 7 bei
Rückbeziehung von Anspruch 3 nur auf Anspruch 2,
d a d u r c h  g e k e n n z e i c h n e t ,
daß eine Klemmleiste mit dübelartigen Vorsprüngen vorgesehen ist, die in entsprechende Löcher an einem unteren
Umfangsflansch des Ringes einsteckbar sind.

9. Vorrichtung nach einem der Ansprüche 1 bis 7 bei Rückbeziehung von Anspruch 3 nur auf Anspruch 2,
d a d u r c h  g e k e n n z e i c h n e t ,

C139861

daß der Ring aus zwei teleskopisch und flüssigkeitsdicht ineinander gesteckten Teilringen besteht, die an ihren unteren Kanten Außenflansche haben.

10. Vorrichtung nach einem der Ansprüche 1 bis 9, d a d u r c h   g e k e n n z e i c h n e t , daß eine Schutzhaube (11) mit einem Befestigungsgurt (12) vorgesehen ist, die die Haube (8) beabstandet umgibt.

0139861

Fig. 1

*Fig.2*

0139861

_Fig.3_

0139861

Fig.5

1

3

Fig.4

1

3

Fig. 6

0139861

Fig. 7
Fig. 8
Fig. 9
Fig. 10
Fig. 11
Fig. 12

Europäisches
Patentamt

**EUROPÄISCHER RECHERCHENBERICHT**

Nummer der Anmeldung

| | **EINSCHLÄGIGE DOKUMENTE** | | EP 84108124.3 | |
|---|---|---|---|---|
| Kategorie | Kennzeichnung des Dokuments mit Angabe, soweit erforderlich, der maßgeblichen Teile | Betrifft Anspruch | **KLASSIFIKATION DER ANMELDUNG (Int. Cl 4)** | |
| A | US - A - 3 042 041 (M.E. JASCALE-VICH) <br> * Gesamt, insbesondere Spalte 1, Zeile 66 - Spalte 2, Zeile 9 * <br> -- | 1 | A 61 M 1/00 | |
| A | US - A - 2 280 915 (J.H. JOHNSON) <br> * Gesamt * <br> -- | 1,7 | | |
| A | GB - A - 114 754 (W.H. THAYLOR) <br> * Gesamt * <br> -- | 1 | | |
| D,A | US - A - 3 026 874 (R.C. STEVENS) <br> * Gesamt * <br> -- | 1 | | |
| D,A | DE - B2 - 2 754 775 (C. HILBER) <br> * Gesamt * <br> ---- | 1 | **RECHERCHIERTE SACHGEBIETE (Int. Cl.4)** <br><br> A 61 M 1/00 <br> A 61 M 7/00 <br> A 61 M 25/00 <br> A 61 M 27/00 | |

Der vorliegende Recherchenbericht wurde für alle Patentansprüche erstellt.

| Recherchenort | Abschlußdatum der Recherche | Prüfer |
|---|---|---|
| WIEN | 14-12-1984 | LUDWIG |